(19) Europäisches Patentamt
European Patent Office
Office européen des brevets

(11) Publication number: 0 498 975 A1

# (12) EUROPEAN PATENT APPLICATION

(21) Application number: **91301079.9**

(51) Int. Cl.[5]: **C07C 43/04**, C07C 41/06

(22) Date of filing: **11.02.91**

(43) Date of publication of application: **19.08.92 Bulletin 92/34**

(84) Designated Contracting States: **DE ES FR GB IT**

(71) Applicant: **TEXACO CHEMICAL COMPANY**
**3040 Post Oak Boulevard**
**Houston, Texas 77056(US)**

(72) Inventor: **Knifton, John Frederick**
**Senior Research Associate, 10900 Catskill Trail**
**Austin, Texas 78750(US)**

(74) Representative: **Brock, Peter William et al**
**UROUHART-DYKES & LORD 91 Wimpole Street**
**London W1M 8AH(GB)**

## (54) Tertiary amyl methyl ether from olefin streams.

(57) C-5 olefins, especially isoamylene, can be converted into t-amyl methyl ether by reaction with methanol, in the presence of modified acidic smectite clay catalysts.

The preferred catalysts are acidic montmorillonite clays whose selectivity has been improved by modification with platinum or palladium, by mixing them with a supported platinum or palladium catalyst, or by reacting them with a heteropoly acid.

EP 0 498 975 A1

This invention concerns an improved method for the synthesis of tertiary amyl methyl ether by the reaction of methanol and a C-5 hydrocarbon stream containing C-5 olefins in the presence of an acidic clay catalyst, particularly a modified montmorillonite acidic silica-alumina clay. The reaction is particularly advantageous in that the tertiary amyl methyl ether (TAME), can be generated continuously in high concentration, e.g. up to 53%, and isolation of TAME by fractional distillation has been demonstrated in a purity as high as 99%.

Currently, there is interest in using smectite clays as catalysts. It is known that the unique structure permits modifications which provide useful catalyst properties.

Some of the unusual properties of smectite clays which make them of interest as catalysts are discussed in an article entitled "Catalysis: Selective Developments", Chem. Systems Report 84-3, 239-249, at Section 3.4320. These compositions are layered, and exhibit a 2:1 relationship between tetrahedral and octahedral sites. In addition, the combination of cation exchange, intercalation, and the fact that the distance between the layers can be adjusted, provide interesting possibilities. In addition, the cations on the interlamellar surfaces ($Ca^{+2}$ or $Na^{+}$) can be replaced with almost any desired cation, by simple ion-exchange techniques.

Various factors affect clay catalysts. In an article entitled "Pillared Clays As Catalysts", in Catal. Rev. Sci. Eng., 30(3), 457-499 (1988), there is a discussion of factors affecting the properties of pillared clays, including methods by which the thermal stability can be improved in the range from 480 to 800°C. It was found that the method used to dry the flocculated clay appeared to be more important in determining the porosity of the final product than the choice of pillaring agent or the clay layer charge. For example, adsorption is one property which varies with drying method. Also, certain chemicals such as $Al_2O_3$ can be fixed on the clays in order to increase surface area by changing the pore size distribution and increasing thermal stability.

The same article also discusses the acidity of pillared clays, as well as ways in which different treatments affect the Lewis or Bronsted sites to a varying extent. It appears to be necessary to invoke the presence of several types of acid sites, because pillared clays do not necessarily function in the same manner as zeolites.

There is a discussion of clay mineral catalysts, including acidic montmorillonite clay catalysts, in "Progress in Inorganic Chemistry", Vol. 35, p. 41 (1987). The process of pillaring this type of catalyst is discussed. Pillaring can convert a clay lamellar solid, from a material that collapses on heating to temperatures of no more than a few hundred degrees Centigrade, into a more heat resistant, two dimensional zeolite-type material that can survive heat treatment in moist atmospheres well above 500°C.

US-A-4176090 and -4248739 disclose the addition of a silicate to an aluminium chlorhydral solution to increase substantially the surface area of clays. Some of the best hydrothermal stability reported to date in the art concerns clay intercalated by hydroxy silica-aluminium ions.

J. Adams, in Applied Clay Science, 2, 309 (1987) provides a review of the use of pillared cation-exchanged and acid-treated montmorillonites as catalysts for reactions which can be carried out on a preparative laboratory or industrial scale. These catalysts are known for displaying Bronsted and Lewis acid activities, but Diels-Alder reactions can also be effected. The ability of the clays to act as Bronsted and Lewis acids is also discussed in "Clays as Selective Catalysts in Organic Synthesis", J. M. Adams and K. Martin, J. of Inclusion Phenomena, 5, 663 (1987) After surveying the Bronsted acid activity of such clays, Adams et al. found that with cation-exchanged clays, the reactions proceeded below 100°C provided they involve tertiary or allylic carbocation intermediates. At 150 to 180°C, reactions involving primary and secondary carbocations are possible.

The same author discusses the fact that potential Lewis acid centres exist in smectite clays. $Al^{3+}$ and $FE^{3+}$ ions are normally associated with the octahedral sheets of the montmorillonite. The reaction of alcohols with isobutene in the presence of montmorillonite catalysts at page 668 is discussed. This reaction gives high yields of the tertiary ether at low temperatures according to the equation:

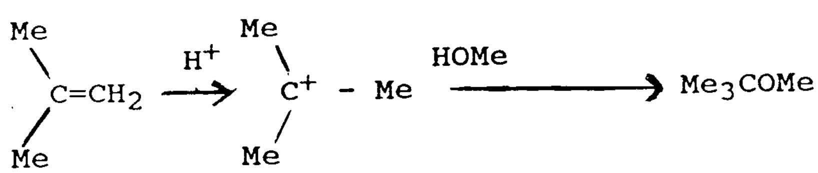


where the rate of reaction was found to be proportional to the isobutene concentration. A plausible reaction

scheme is as follows:

$$A(l) \overset{K_1}{\rightleftarrows} A(i) \qquad \text{where } l = \text{liquid phase}$$

$$MeOH(l) \overset{K_2}{\rightleftarrows} MeOH(i) \qquad i = \text{intercalated species}$$

$$MeOH(i)+H^+ \overset{K_3}{\rightleftarrows} MeOH_2(i) \qquad A = \text{alkene}$$

$$MeOH_2(i)+A(i) \overset{K_4}{\rightleftarrows} MeOH(i)+AH^+(i)$$

---

$$MeOH(i)+AH^+(i) \overset{K_5}{\rightleftarrows} MTBE(i) + H^+$$

$$MTBE(i) \overset{K_6}{\rightleftarrows} MTBE(l)$$

The rate determining step appears to be protonation of the alkene (K4). Apparently solvent effects can be great, with the use of 1,4-dioxane increasing the rate six-fold and promoting miscibility of all reagents. In contrast, the reaction of alcohols with linear alk-1-enes is slow and give mixtures of alk-2-yl and alk-3-yl ethers.

The use of Group IV Metal Phosphates is discussed in "Recent Advances in Pillared Clays and Group IV Metal Phosphates", A. Clearfield, Surface Organometallic Chemistry; Molecular Approaches to Surface Catalysis, 231, 271 (1988). It is known that smectite clays swell with water and are able to exchange large cations, such as $[A1_{13}O_4(OH)_{24}\text{-}12H_2O]^{7+}$ and $[Zr(OH)_2{\cdot}4H_2O]_4^{8+}$. These large cations can then act as pillars to prop open the clay layers. This property permits the creation of catalyst materials with pores larger than those found in zeolites. In particular there is disclosed the potential use of the layered compound $\alpha$-zirconium phosphate, which is pillared by cross-linking the layers with aryl diphosphonic acid. Where the clay is pillared, the pillar spacing apparently is determined by the radius of the ingoing cation.

Smectites characteristically have alumina octahedra sandwiched between layers of silicate tetrahedra. In smectites, substitutions of $Mg^{2+}$ for $Al^{+3}$ and $Li^{+}$ for $Mg^{2+}$ can take place at the octahedral site, or $M^{3+}$ ions can substitute for $Si^{4+}$ at the tetrahedral site.

In J. Am. Chem. Soc., 101, 6891 (1979), Pinnavaia et al. pointed out that the rapid tumbling of simple intercalated ions, such as hydrated $Cu^{2+}$ and $Mn^{2+}$, and movement of free solvent in the clay interlayers, indicate the possibility of carrying out metal complex-catalyzed reactions in the intra-crystal space of said minerals.

In an article by S. Randriamahefa, et al., J. Mol. Catal., 49, 85 (1988) entitled "Synthesis de L'Ether Methyl Ter-Amylique (TAME) En Catalyse Acide: Cinetiques Et Equilibres De La Methoxylation du Methyl-2-Butene-2", there is a discussion of the preparation of tertiary amyl methyl ether by methoxylation of a pure $C_5$ isoolefin using a catalyst comprising a polymer-supported sulfonic acid.

An article by Y. V. Subba, et al., J. Mol. Catal., 49, L47 (1989), describes for the first time the synthesis and characterization of interlamellar montmorillonite 3-aminopropyltriethoxysilane (IA) and its palladium(II) complex, and the selective reduction of aromatic carbonyls only to the corresponding carbinols, and of nitrobenzene to aniline, at room temperature and atmospheric pressure. Selectivity in the hydrogenation of carbonyls is demonstrated, and is ascribed to the change in electronic and coordination environment of the complex brought about in the interlayers of montmorillonite. Tests indicated that the palladium in the used catalyst remains in the divalent state. The catalyst displayed less than 1% of its original hydrogenation activity after the extractive treatment, and the results demonstrate that hydrogenation has not been effected by a route involving metallic Pd particles.

In an article entitled "A Novel Anchored" Palladium(II) Phosphinated Montmorillonite: the First Example in the Interlamellars of Smectite Clay", J. Chem. Soc. Chem Commun., 931, (1985), B. M. Choudary, et al. disclose that montmorillonite containing $PdCl_2$ anchored by pendant phosphinated groups in the interlamellar region, has been found to hydrogenate selectively terminal alkenes and alkynes. This work suggests that sterically hindered double and triple bonds could be selectively reduced by suitable control of interlayer expansion.

The synthesis of methyl t-butyl ether from methanol and isobutene using a clay catalyst is featured in another article by J. M. Adams et al. in Clay And Clay Minerals, 30, 129 (1982).

US-A-4605806 and -4665220 disclose that layered clays such as montmorillonites are useful as catalysts, for instance, in the etherification of olefins with alkanols. Synthesis of tertiary amyl methyl ether by rapid conversion of methanol and a C-5 olefin over an improved acidic clay, would have significant commercial value. Such a process would be particularly efficient if TAME were manufactured as a primary or secondary product from typical C-5 raffinate streams from the production of MTBE, butadiene and light olefins. Furthermore the acidic clay catalysts described are particulary efficient and thermally stable.

This invention concerns a process for preparing tertiary amyl methyl ether by reacting methanol and a C-5 olefin over an acidic clay catalyst modified by palladium, or a heteropoly acid, particularly a montmorillonite acidic silica-alumina clay, at a temperature of 20 to 250°C and a pressure of 0.1 to 7.0 MPa.

In various embodiments of the invention, the clay is modified by:

(i) bonding a heteropoly acid to the clay,

(ii) modification of the clay with platinum or palladium, and

(iii) mixing the clay with a supported platinum or palladium catalyst.

The catalyst preferably comprises a montmorillonite acidic silica-alumina clay in powdered, granular or extruded form.

A particular advantage of this invention over the prior art is that C-5 raffinate streams from MTBE, butadiene and light olefins can be used to produce TAME, which is useful as an octane enhancer for refiners and in gasoline. The TAME can be isolated in a purity as high as 99% purity by fractional distillation.

The reaction can be represented by the equation:

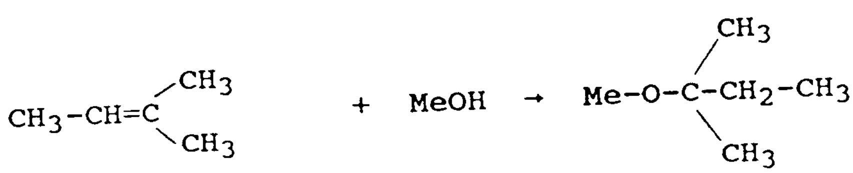

(Eq. 1)

The method is most useful in the reaction of C-5 olefins, particularly those containing a significant quantity of isoamylene. The same method may also be applied to the reaction of other C-5 olefins. For example, the process may be applied to the reaction of a low molecular weight alkanol with 2-methyl-1-butene, 2-methyl-2-butene, or 3-methyl-1-butene.

The modified catalysts used to effect this reaction are preferably obtained by modifying silica-and alumina-rich montmorillonite acidic clay catalysts. A variety of clay catalysts containing aluminium and silica are effective in the reaction (Eq.1), but it is necessary that the alumina or silica should be acidic under normal operating conditions. Chemically clays are composed primarily of silicon, aluminium and oxygen, with minor amounts of magnesium and iron in some instances. Variations in the ratios of these constituents, and in their crystal lattice configurations, result in some fifty separate clays, each with its own characteristic properties.

Particularly effective in the reaction of Equation 1 are the smectite clays used according to the invention. Smectite clays are discussed in the article cited in Chem. Systems Report, 84-3. These clays have small particle size and unusual intercalation properties, which give them high surface area. They are aluminosilicates with a unique structure that permits modifications which provide useful catalysts. They comprise layered sheets of octahedral sites between sheets of tetrahedral sites. The distance between the layers can be adjusted by swelling, by treatment with an appropriate solvent, or by treatment with a pillaring or Lewis acid reagent etc. What renders the smectites of particular interest among the clay minerals, is their combination of cation exchange, intercalation and swelling properties.

The three-layer sheet types of smectite clays include montmorillonite, vermiculite and certain micas, all of which may be expanded between their layers by appropriate treatment. The idealized basic structure of clays of this type is that of a pyrophyllite, which has the basic formula $Si_8Al_4O_{20}(OH)_4$.

A general representation of the montmorillonite structure is:

$$M_{x/n}^{n+} \bullet yH_2O(Al_{4-x}Mg_x)(Si_8)O_{20}(OH)_4$$

where M represents the interlamellar (balancing cation), normally sodium or lithium and x, y and n are numbers.

These montmorillonite clays are best used in the present application in an acidic form. Acids activate montmorillonites by attacking and solubilizing structural cations in the octahedral layers. This opens up the clay structure and increases surface area. These acid treated clays act as strong Bronsted acids.

Acidic montmorillonite clays are the preferred form of smectite clay for use in the present invention. They may be acidified by pretreatment with mineral acid, e.g. sulphuric acid or phosphoric acid. Preferably these acid clays should have acidities of 3 to 20, mg KOH/gm, titrated to a phenolphthalein end point, but may have higher acidity. Their surface area should be above 30 $m^2/g$, and preferably 200 to 1000 $m^2/g$. Their moisture content should be limited also; preferably the weight loss is less than 20 wt% on heating at 99-100°C.

Illustrative examples of suitable acidic montmorillonite clays include Engelhard's powdered Clay-113, having a residual acidity of 10 mg KOH/gm, a surface area of 300 $m^2/gm$ and a moisture content of 4 wt%, Clay-13, having an acidity of 16 mg KOH/gm, a surface area of 300 $m^2/gm$ and a moisture content of 16 wt%; granular Clay-24 of particle size 20/60 mesh (0.25-0.84 mm) having an acidity of 16 mg KOH/gm, a surface area of 300 $m^2/gm$ and a moisture content of 10 wt%; granular Clay-25, of particle size 10/20 mesh (0.84-2.0 mm) having an acidity of 16 mg KOH/gm, a surface area of 400 $m^2/gm$ and a moisture content of 12 wt%; granular Clay-224, of particle size 20/60 mesh (0.25-0.84 mm) having an acidity of 3.0 mg XOH/gm, a surface area of 350 $m^2/gm$ and a moisture content of <1 wt%; and extruded Clay-62, which may, for example, be in 1/16" (1.59 mm) or 3/16" (4.76 mm) diameter extrudates, and have acidity of ca. 3.0 mg KOH/gm, a surface area of 275 $m^2/gm$ and a moisture content of less than 1%.

In one embodiment of the invention, a palladium catalyst, such as a palladium salt, oxide, or complex can be exchanged into the clay, or chemically bonded to the structure of the inorganic support, in order to achieve improved selectivities and yields of desired tertiary amyl methyl ether, particularly from mixed C-5 olefin feedstocks. Suitable palladium derivatives include such palladium salts as palladium chloride, actylacetonate and acetate. The palladium salts may be introduced into the clay by an ion exchange process, or they may be chemically bonded to a functionalized clay, e.g. an aminosilated clay, such as an aminosilated Engelhard Clay-24 prepared according to the method of Choudary et. al. J. Mol. Catal., 49, L47 (1989), which describes the preparation of an interlamellar montmorillonite-silylamine-palladium(II) catalyst.

An additional possibility is the use of the classes of acidic clay outlined above in combination with a separate heterogeneous palladium catalyst. In particular, there is the possibility of employing an acidic clay catalyst in combination with a separate supported palladium catalyst, for example, a palladium-on-carbon catalyst. Here the support may be carbon, alumina, silica, titania, zirconia, magnesia, as well as combinations thereof. The palladium loading should be from 0.1 to 20 wt%. Suitable examples include the use of a combination of Engelhard Clay-24 granules with a 5% palladium-on-carbon catalyst. These catalyst combinations may be intimately mixed, or they may be used separately; the weight ratios may vary anywhere from 100:1 to 1:100 or clay-to-Pd catalyst.

In a further embodiment of this invention, a heteropoly acid is bonded to the clay catalyst, in order to achieve improved selectivities and yields of desired tertiary amyl methyl ether particularly from mixed C-5 olefin feedstocks. Suitable heteropoly acids which can be bonded to the clay comprise a class of acids formed by the condensation of two or more inorganic oxyacids. For example, phosphate and tungstate ions, when reacted in an acidic medium, are condensed to form 12-tungstophosphoric acid, a typical heteropoly acid (HPA) according to Equation 2:

$$PO_4^{3-} + 12WO_4^{2-} + 27H^+ \text{------>} H_3PW_{12}O_{40} + 12H_2O \qquad \text{(Eq. 2)}$$

A wide variety of elements ranging from Group I to Group VIII can become the central atom of the HPA anion, or the heteroatom as it is called (P in the case of Eq. 2). The nature of the heteroatom is a governing factor which determines both the condensation structure and the physical properties of the HPA.

Atoms coordinated to the heteroatom via oxygens are called polyatoms (W in the case of Equation 2)

and in most instances are any one of such limited species as molybdenum, tungsten, niobium or vanadium. The nature of the heteroatoms, condensation ratios and chemical formulae of the corresponding HPA anions are summarized below for the case of molybdenum (Mo) polyatom.

Anions containing the so-called Keggin structure have a condensation ratio of 1:12 and are the most typical HPA anions. Heteropoly acids with the Keggin structure, and their homologues, are generally the most readily available and the most commonly used in catalysis. The synthesis of these heteropolyacids is well documented in the literature (see for example US-A-3947332).

## TYPICAL HETEROPOLYMOLYBDATE ANIONS

| CONDENSATION RATIOS | | HETERO ATOMS(X) | CHEMICAL FORMULAS |
|---|---|---|---|
| 1:12 | Keggin structure | $P^{5+}$, $As^{5+}$, $Si^{4+}$, $Ge^{4+}$ | $[X^{n+}Mo_{12}O_{40}]^{-(8-n)}$ |
| | Silverton structure | $Ce^{4+}$, $Th^{4+}$ | $[X^{4+}Mo_{12}O_{42}]^{8-}$ |
| 1:11 | Keggin structure (decomposition) | $P^{5+}$, $As^{5+}$, $Ge^{4+}$, $Si^{4+}$ | $[X^{n+}Mo_{11}O_{39}]^{-(12-n)}$ |
| 2:18 | Dawson structure | $P^{5+}$, $As^{5+}$ | $[X_2^{5+}Mo_{18}O_{62}]^{6-}$ |
| 1:9 | Waugh structure | $Mn^{4+}$, $Ni^{4+}$ | $[X^{4+}Mo_9O_{32}]^{6-}$ |
| 1:6 | Anderson structure (A type) | $Te^{6+}$, $I^{7+}$ | $[X^{n+}Mo_6O_{24}]^{-(12-n)}$ |
| | (B type) | $Co^{3+}$, $Al^{3+}$, $Cr^{3+}$ | $[X^{n+}Mo_6O_{24}H_6]^{-(6-n)}$ |
| 4:12 | | $As^{5+}$ | $[H_4As_4Mo_{12}O_{52}]^{4-}$ |
| 2:5 | | $P^{5+}$ | $[P_2Mo_5O_{23}]^{6-}$ |

Suitable heteropoly acid catalysts for use according to the present invention may contain molybdenum, tungsten, niobium or vanadium as polyatom, and phosphorus, silicon, germanium or arsenic as heteroatom. Preferably the heteroatom is phosphorus or silicon. These heteropoly acids would probably have the Keggin structure,$H_{8-n}[XM_{12}O_{40}]$, where x = P or Si, M = Mo or W, and n is 4 or 5.

The preferred heteropoly acids for the practice of this invention include 12-molybdophosphoric acid, $M_3PMo_{12}O_{40}$, 12-tungstophosphoric acid, molybdosilicic acid, $H_4SiMo_{12}O_{40}$and 12-tungstosilicic acid. Said acids are generally used as their hydrates; they may be employed by themselves, partially or completely dissolved in the feed, or preferably as heterogeneous catalysts bonded to a suitable clay support.

The wt% concentration of the polyatom, M, in the formulated heteropoly acid-on-clay catalyst should generally be from 0.1 to 20 wt%.

The above described types of catalyst could also be used for the cogeneration of MTBE, TAME and higher analogues. Two additional results of using these classes of catalyst might be:

a) The hydrogenation to monoolefins of any diolefin present in the feed stream.

b) Isomerization of the double bonds of the monoolefin fractions of the same feedstock.

Preparation of tertiary amyl methyl ether may be conducted batchwise, in a continuous slurry bed reactor, or in a fixed-bed, continuous flow, reactor. For practical reasons a fixed bed process is preferred. The catalyst or catalyst combination is effective in the form of a powder, extrudate or granules. In all instances the amount of catalyst should be sufficient to provide the desired catalytic effect.

Preparation of TAME can generally be conducted at temperatures from 20 to 250°C (or 20 to 200°C when the catalyst modified with heteropolyacid is employed); the preferred range is 60 to 180°C. The operating pressure may be from 0.1 to 7.0 MPa (0 to 1000 psig) or even higher. The preferred pressure range is from 0.8 to 2.9 MPa (100 to 400 psig).

Typically, TAME is generated continuously in up to 53 wt% concentration or higher, in the crude product liquid effluent. The TAME can be isolated in up to 99% purity by fractional distillation. The olefin feedstock component may be pure isoamylene, a mixture of C-5 olefins (such as a mixture of 2-methyl-1-butene, 2-methyl-2-butene and 3-methyl-1-butene), a mixture of C-5 olefins and C-5 alkanes, including n-pentane and iso-pentane, or a C-5 distillation cut that may comprise hydrocarbons with 2, 3, 4, 5, 6 or more carbon atoms per molecule. The C-5 cut may contain saturated hydrocarbons such as isobutane, n-butane, isopentane, hexanes etc., in combination with olefins, such as 1-butene, cis-and trans-2-butene, isobutylene, isoamylene, 3-methyl-1-butene, and 2-methyl-1-butene, plus polyolefins such as 1,3-butadiene and isoprene. Typical feedstocks include a C-5 raffinate stream from an MTBE unit and a C-5 stream from a light olefins unit. Such feedstocks may contain I to 50% isoamylene.

The alcohol/C-5 olefin feed can also be diluted with a suitable solvent before being fed to the reactor. Suitable diluents include saturated hydrocarbons such as n-pentane, and ether solvents such as p-dioxane.

The alcohol to C-5 olefin feed molar ratio may be from 100:1 to 1:100, but generally a small excess of alcohol is employed. The preferred methanol-to-isoamylene feed molar ratio is 1:1 to 10:1.

Generally methanol and C-5 olefin conversions are good, and TAME selectivities, based upon either methanol or isoamylene converted, are often 93-99 mole% or higher. When the C-5 feedstock is a mixture of C-5 olefins, the TAME selectivities, based on isoamylene, may exceed 100%, because other C-5 olefin fractions, particularly the 2-methyl-1-butene, isomerize and react with the methanol to form desired product. These selectivities are achieved at total liquid hourly space velocities of 1-10, or higher. Here LHSV is defined as:

$$\text{LHSV} = \frac{\text{Weight Of Total Liquid Feed Run Through The Reactor Per Hour}}{\text{Volume of Catalyst In Reactor}}$$

The Examples which follow illustrate the preparation of TAME from methanol with a) pure isoamylene, b) isoamylene diluted with n-pentane and c) a C-5 raffinate stream containing various C-5 olefins including isoamylene. Suitable catalysts for these TAME syntheses illustrated below include:

1) acidic montmorillonite clays.

2) an acidic montmorillonite clay catalyst containing exchanged palladium salt.

3) a montmorillonite-silylamine-palladium (II) catalyst.

4) a physical mixture of montmorillonite clay plus supported palladium catalyst.

5) acidic silica-alumina clays having heteropoly acids, such as 12-tungstophosphoric acid, bonded to them.

Conversion of methanol, or isoamylene, into TAME is estimated in the following Examples using equations of the type:

$$\frac{[\text{wt\% conc of isoamylene in feed} - \text{wt \% conc of isoamylene in product}] \times 100}{[\text{wt\% conc of isoamylene in feed}]}$$

Selectivities to TAME are generally estimated from:

$$\frac{\text{Moles of TAME in Product}}{\text{Moles of Methanol (or Isoamylene) Converted}}$$

The Examples, including tabular data, illustrate the one-step synthesis of TAME from C-5 olefin plus methanol. Points which should be noted in the following Examples include:

a) In Example 1, Englehard Clay-24 catalyst was employed to give TAME in up to 27% concentration when run at an LHSV of 1 to 10 and a temperature from 80 to 140°C. The feed mix comprised pure methanol plus isoamylene in an approximately 2:1 weight ratio (4.8:1 molar ratio). b) When the methanol: isoamylene molar feed ratio is 1.2:1, as in Example 2, TAME may form up to 53% of the product effluent, in 93-99 mole% yields (basis methanol and isoamylene converted respectively). The TAME may be isolated in 98% purity by fractional distillation.

c) When the same 1.2:1 molar mixture of methanol/isoamylene is diluted with n-pentane, TAME is again generated in good yields (see Example 3).

d) TAME production has also been demonstrated when the feedstock is a C-5 raffinate stream containing 6.9% of isoamylene (see Example 4).

e) TAME production is similarly demonstrated when the feedstock is a C-5 cut containing 14.3% of isoamylene from a light olefins unit (Example 5). In this Example, the TAME selectivity exceeds 100 mol%, indicating that other C-5 olefin components in the feed mixture are being etherified to give TAME.

f) TAME production is also demonstrated with a palladium-modified montmorillonite clay. Selectivities to

TAME are very good (Example 6).

g) Selectivity to TAME is further raised to 264 mole% (Basis apparent isoamylene conversion) when the catalyst is a mixture of acidic montmorillonite clay and 5% palladium-on-carbon (Example 7, compare with Example 5). This result indicates that even more of the other C-5 olefin components in the feed are being etherified to give the desired TAME.

h) When the 1.2:1 molar mixture of methanol and isoamylene is diluted with n-pentane (Example 8), a palladium-on-aminosilanated clay also generates TAME.

i) In Example 9, the TAME selectivity significantly exceeds 100 mole%, basis isoamylene converted, indicating that other C-5 olefin components in the feed mixture are being etherified to give tertiary amyl methyl ether.

j) Selectivity to TAME is further raised to 261 mole% (basis apparent isoamylene converted) when the catalyst is a 12-tungstophosphoric acid modified clay extrudate (see Example 10). This result indicates that still more of the other C-5 olefin components in this feed are being etherified to give the desired TAME.

Example 1 (for Comparison)

The synthesis of t-amyl methyl ether from isoamylene and methanol using an acidic clay catalyst, was conducted in a tubular reactor with an internal diameter of 14.3 mm (0.563 inch) and a length of 25.4 mm (12 inch), constructed of 316 stainless steel, operated upflow and mounted in a furnace, controllable to ±1.0°C and fitted with pumps allowing flow control to less than ±1 cc/h. The reactor was also fitted with a pressure-regulating device and equipment for monitoring temperature, pressure and flow rate.

The reactor was charged with 25 cc of Engelhard Clay-24 granules. A screen of glass beads was placed at the top and bottom of the reactor to ensure that the clay granules would remain in the middle portion.

The catalyst bed was first conditioned overnight by washing with a methanol/isoamylene (2:1) mixture at 100°C, 2.17 MPa (300 psi) back pressure and a liquid flow rate of 25 cc/h. The same solution of methanol + isoamylene (2:1, weight ratio) was then pumped through the catalyst bed at 25 cc/h, while the reactor was held at 100°C and a total pressure of 2.17 MPa (300 psi). Samples of the product were taken periodically, by collecting on-stream in a 316 stainless steel pressure bomb. Typical analysis data for samples taken under these conditions are summarized in Table I.

Catalyst performance over a range of temperatures (80-140°C) and liquid flow rates (25-250 cc/h. LHSV 1-10) was also measured, after reaching equilibrium conditions. Summary data for these additional six runs are also given in Table I.

A sample of the composite effluent (1744g) was fractionally distilled at atmospheric pressure. A distillate fraction (233g) boiling at 85-86°C comprises >96% tertiary amyl methyl ether (TAME).

TABLE I

Catalyst: Clay-24
Feed % : MeOH 68.7; $C_5H_{10}$ - 31.2

| Temp. (°C) | Flow (cc/h) | Sample | ← PRODUCT COMPOSITION (%) → MeOH | $C_5OH$ | $C_5$= | TAME | Day |
|---|---|---|---|---|---|---|---|
| 100 | 25 | 1 | 66.4 | 11.6 | 0.2 | 21.7 | 1 |
| | | 2 | 59.1 | 13.3 | 0.1 | 27.4 | 2 |
| | | 3 | 58.7 | 13.5 | 0.1 | 27.8 | |
| 120 | 25 | 4 | 59.2 | 14.1 | 0.1 | 26.5 | 3 |
| | | 5 | 58.9 | 14.1 | 0.3 | 26.5 | |
| | | 6 | 59.6 | 13.9 | 0.1 | 26.3 | 4 |
| | | 7 | 59.0 | 14.2 | 0.1 | 26.6 | |
| 140 | 25 | 8 | 60.4 | 17.7 | 0.1 | 21.0 | 5 |
| | | 9 | 60.4 | 17.6 | 0.1 | 21.0 | |
| | | 10 | 60.6 | 17.7 | 0.1 | 20.7 | 6 |
| | | 11 | 60.4 | 17.6 | 0.1 | 21.1 | |
| 80 | 25 | 12 | 62.8 | 21.0 | 0.1 | 16.1 | 7 |
| | | 13 | 62.8 | 21.0 | 0.1 | 16.2 | |
| | | 14 | 63.5 | 19.9 | 0.1 | 16.4 | 8 |
| | | 15 | 62.8 | 21.2 | 0.1 | 15.9 | |
| 100 | 25 | 16 | 59.8 | 13.7 | 0.1 | 26.4 | 9 |
| | | 17 | 58.9 | 13.8 | 0.1 | 27.2 | |
| 100 | 100 | 18 | 60.0 | 16.7 | | 23.2 | 10 |
| | | 19 | 60.1 | 16.8 | 0.1 | 23.0 | |
| 100 | 250 | 20 | 62.8 | 22.1 | 0.1 | 15.1 | 11 |
| | | 21 | 62.8 | 22.2 | 0.1 | 14.9 | |

Example 2 (for Comparison)

Following the procedures of Example 1, a fresh sample of Clay-24 granular catalyst was treated with a 1.2:1 molar mixture of methanol (768g) and isoamylene (1402g) at 100°C using a flow rate of 25 cc/h. Samples of the product effluent were taken on-stream periodically and analyzed by GLC. Results are summarized in Table II.

Again catalyst performance was investigated over a range of temperatures (80-140°C) and liquid flow rates (25-100 cc/h). Summary data for these additional runs are also given in Table II.

A sample of the composite effluent (989g) was fractionally distilled at atmospheric pressure. A distillate fraction (127g) boiling at ca. 85°C comprised 98% TAME.

An analysis of the data from Sample 4 shows:

| | |
|---|---|
| Isoamylene conversion | 57% |
| Methanol conversion | 48% |
| TAME selectivity (basis isoamylene) | >99 mole% |
| TAME selectivity (basis methanol) | 93 mole% |

## TABLE II

Catalyst: Clay-24
Feed % : MeOH 36.8; $C_5H_{10}$ - 63.1

| Temp. (°C) | (cc/h) | Sample | PRODUCT COMPOSITION (%) MeOH | $C_5H_{10}$ | TAME |
|---|---|---|---|---|---|
| 100 | 25 | 1 | 23.8 | 26.5 | 48.7 |
| | | 2 | 20.7 | 27.8 | 50.2 |
| | | 3 | 19.8 | 27.0 | 52.8 |
| | | → 4 | 19.0 | 27.1 | 53.6 |
| 120 | 25 | 5 | 22.7 | 32.7 | 44.1 |
| | | 6 | 22.2 | 33.8 | 43.8 |
| 140 | 25 | 7 | 25.5 | 39.4 | 34.1 |
| | | 8 | 25.5 | 40.3 | 31.5 |
| 80 | 25 | 9 | 25.4 | 38.1 | 35.7 |
| | | 10 | 23.6 | 39.7 | 36.7 |
| 100 | 100 | 11 | 20.0 | 29.7 | 50.3 |
| | | 12 | 20.4 | 30.6 | 49.0 |

EXAMPLE 3 (for Comparison)

Following the procedures of Example 1, a fresh sample of Clay-24 granular catalyst (40 cc) was treated with a 2:1 molar mixture of methanol/isoamylene diluted with n-pentane at 80°Cusing a flow rate of 80 cc/h. Samples of the product effluent were taken on-stream periodically and analyzed by GLC. Results are summarized in Table III.

Again catalyst performance was investigated over a range of temperatures (60-120°C). Summary data for these additional runs are also given in Table III.

An analysis of the data from Sample 5 shows:

| | |
|---|---|
| Isoamylene conversion | 51% |
| Methanol conversion | 39% |
| TAME selectivity (basis methanol) | 95 mole% |

## TABLE III

Catalyst: Clay-24
Feed%: MeOH 10.0; $C_5H_{10}$ 17.7; $C_5H_{12}$ 71.4.

| Temp. (°C) | Flow (cc/h) | Sample | PRODUCT COMPOSITION (%) MeOH | $C_5H_{10}$ | $C_5H_{12}$ | TAME |
|---|---|---|---|---|---|---|
| 80 | 80 | 1 | 8.1 | 13.6 | 70.7 | 5.9 |
| | | 2 | 8.1 | 13.7 | 71.1 | 5.4 |
| | | 3 | 7.9 | 13.3 | 71.1 | 6.0 |
| | | 4 | 7.3 | 14.0 | 71.7 | 5.2 |
| 100 | 80 → | 5 | 6.1 | 8.6 | 71.0 | 11.8 |
| | | 6 | 6.1 | 8.8 | 71.1 | 11.6 |
| 120 | 80 | 7 | 6.4 | 10.5 | 71.0 | 8.8 |
| | | 8 | 6.9 | 10.6 | 71.0 | 8.8 |
| 60 | 80 | 9 | 9.7 | 17.6 | 71.2 | 0.7 |
| | | 10 | 9.8 | 17.4 | 71.3 | 0.7 |

Example 4 (for Comparison)

Following the procedures of Example 1, a fresh sample of granular Clay-24 catalyst (40 cc) was treated with a mixture of C-5 raffinate (3000g) from a MTBE unit, containing about 6.9% isoamylene, and methanol (320g) at 80°C, using a flow rate of 80 cc/h. Samples of the product effluent were taken on-stream periodically and analyzed by GLC for C-5 and TAME content. Results are summarized in Table IV.

Catalyst performance was checked over a range of operative temperatures (80-120°C) and flow rates (40-80 cc/h). Summary data for these additional runs may also be found in Table IV.

## TABLE IV

Catalyst: Clay-24

| Temp. (°C) | Flow (cc/h) | Sample | Liquid Composition (%) C-4 Olefins 1- | ISO- | CIS-2 | TRANS-2 | 3-Me-1 | 2-Me-2 | Total Sample MeOH | TAME |
|---|---|---|---|---|---|---|---|---|---|---|
| | | F | 2.7 | 0.2 | 3.2 | | 2.5 | 6.9 | 8.3 | 0.3 |
| 80 | 80 | 1 | 0.6 | | 3.2 | 2.7 | 2.5 | 6.5 | 6.5 | 1.0 |
| | | 2 | 0.6 | 0.1 | 3.3 | 2.8 | 2.5 | 5.7 | 8.4 | 1.5 |
| 100 | 80 | 3 | 2.7 | 0.1 | 3.3 | | 2.5 | 4.7 | 7.4 | 3.6 |
| | | 4 | 2.8 | 0.1 | 3.3 | | 2.6 | 4.8 | 7.6 | 3.9 |
| 120 | 80 | 5 | 0.6 | 0.2 | 3.1 | | 2.5 | 4.9 | 7.7 | 2.4 |
| | | 6 | 0.6 | 0.3 | 3.1 | | 2.4 | 5.1 | 7.4 | 2.2 |
| 100 | 40 | 7 | 2.6 | 0.1 | 3.1 | | 2.5 | 4.7 | 8.6 | 3.8 |
| | | 8 | 2.7 | 0.1 | 3.2 | | 2.5 | 4.7 | 8.6 | 3.8 |
| 120 | 40 | 9 | 0.6 | 0.2 | 3.0 | 2.6 | 2.4 | 5.0 | 8.6 | 2.7 |
| | | 10 | 0.6 | 0.2 | 3.1 | 2.6 | 2.4 | 5.0 | 8.2 | 2.6 |

Example 5 (for Comparison)

Following the procedures of Example 1, a fresh sample of granular Clay-24 catalyst (40cc) was treated with a mixture of C-5 cut (3000g) from a light olefin unit, containing about 14.3% isoamylene, plus methanol

(640g) at 80°C using a flow rate of 80 cc/h. Samples of the product effluent were taken on-stream periodically and analyzed by GLC, GLC-ir and GLC-m.s. for C-5 and TAME content. Results are summarized in Table V.

Again catalyst performance was checked over a range of operating temperatures (80-120°C) and flow rates (40-80 cc/h).

An analysis of the data from Sample 3 shows:

| | |
|---|---|
| Apparent isoamylene conversion | 43% |
| Methanol conversion | 51% |
| TAME selectivity (basis isoamylene) | 124 mole% |

## TABLE V

Catalyst Clay-24

| | | | Liquid Composition (%) | | | | | | | | | |
|---|---|---|---|---|---|---|---|---|---|---|---|---|
| | | | C-4 Olefins | | | | | C-5 Fraction | | | Total Sample | |
| Temp. (°C) | Flow (cc/h) | Sample | 1- | ISO- | CIS-2 | TRANS-2 | $C_4^{2-}$ | 3-Me | 2-Me-2 | 2-Me-1 | MeOH | TAME |
| | | F-1 | 0.9 | 0.6 | 3.0 | 5.3 | 6.7 | 30.2 | 14.3 | 4.0 | 22.4 | - |
| 80 | 80 | 1 | 0.9 | 0.2 | 3.2 | 5.6 | 7.4 | 32.8 | 9.4 | 1.3 | 14.2 | 7.2 |
| | | 2 | 0.9 | 0.1 | 3.3 | 5.7 | 7.4 | 32.8 | 9.1 | 1.2 | 12.7 | 7.5 |
| 100 | 80 | → 3 | 0.9 | 0.1 | 3.3 | 5.8 | 7.7 | 33.9 | 8.1 | 1.2 | 10.9 | 8.8 |
| | | 4 | 0.9 | 0.1 | 3.2 | 5.6 | 7.4 | 32.7 | 7.9 | 1.2 | 11.6 | 8.7 |
| 120 | 80 | 5 | 0.9 | 0.1 | 3.3 | 5.7 | 7.7 | 32.7 | 9.6 | 1.8 | 12.0 | 6.2 |
| | | 6 | 0.9 | 0.2 | 3.3 | 5.7 | 7.7 | 32.6 | 9.5 | 1.8 | 12.0 | 6.2 |
| 100 | 40 | 7 | 1.0 | 0.1 | 3.5 | 5.9 | 7.8 | 33.7 | 8.3 | 1.2 | 12.0 | 8.7 |
| | | 8 | 1.0 | 0.1 | 3.5 | 6.0 | 7.7 | 33.4 | 8.2 | 1.2 | 11.4 | 8.7 |
| 120 | 40 | 9 | 1.0 | 0.2 | 3.4 | 6.0 | 7.8 | 32.1 | 10.0 | 1.7 | 15.2 | 7.0 |
| | | 10 | 1.0 | 0.1 | 3.4 | 6.0 | 7.8 | 32.0 | 10.0 | 1.7 | 15.4 | 7.2 |
| 80 | 80 | 11 | 1.0 | 0.2 | 3.3 | 5.9 | 7.5 | 31.0 | 10.6 | 1.9 | 13.7 | 5.6 |
| | | 12 | 1.0 | 0.2 | 3.3 | 6.0 | 7.5 | 31.0 | 10.6 | 1.9 | 13.4 | 5.8 |

The following Examples A and B describe the preparation of palladium-modified catalyst for use in Examples 6 and 8 according to the invention.

### EXAMPLE A

To a 50g sample of Engelhard Clay-24 granules was added a solution of palladium acetylacetonate (1.5g 5mmole) in absolute ethanol (200 cc). The mixture was stirred slowly at 55°C for 6 h and filtered, and the solids were washed with ethanol and dried overnight in vacuo at 40°C.

The recovered grey solids (48g) were found to contain 0.2% Pd.

### EXAMPLE B

To a 1 litre flask was added 11.5g of dichlorobis (benzonitrile) palladium(II) (30 mmole), 100g of aminosilanated Clay-24 (prepared according to the literature method) and 500g of dried toluene. The mixture was stirred at ambient temperature for 24 hours and filtered, and the solids were first washed with dried toluene then extracted with toluene for 8 h. The filtered solids were again washed with toluene and

dried *in vacuo* at 40°C.

The recovered grey solids (108g) were found to contain 2.7% Pd and 1.2% N.

EXAMPLE 6

Following the procedures of Example 1, a sample of palladium-modified montmorillonite clay (25cc) prepared according to the procedure of Example A, was treated with a mixture of methanol and isoamylene over a range of operating temperatures (80-120°C) and flow rates (25-200cc/h, LHSV = 1-8). Samples of the product effluent were taken on-stream periodically and analyzed by GLC. Results are summarized in Table VI.

An analysis of the data from Sample 2 shows:

| | |
|---|---|
| Concentration of TAME in the crude product effluent | 53% |
| TAME selectivity (basis methanol) | 90 mole% |
| TAME selectivity (basis isoamylene) | >99 mole% |

TABLE VI

Feed%: MeOH 38.2; $C_5H_{10}$ 61.7.

| Temp. (°C) | Flow (cc/h) | Sample | ←PRODUCT COMPOSITION (%)→ MeOH | $C_5H_{10}$ | TAME |
|---|---|---|---|---|---|
| 100 | 25 | 1 | 19.7 | 28.4 | 50.9 |
| | | → 2 | 19.6 | 27.4 | 53.0 |
| | | 3 | 19.1 | 27.6 | 53.1 |
| 80 | 25 | 4 | 24.3 | 37.7 | 37.9 |
| | | 5 | 24.3 | 38.2 | 37.5 |
| 120 | 25 | 6 | 46.6 | 23.8 | 29.6 |
| | | 7 | 23.6 | 34.1 | 41.8 |
| 100 | 100 | 8 | 21.9 | 31.5 | 46.7 |
| | | 9 | 21.9 | 31.8 | 46.2 |
| | 200 | 10 | 24.1 | 36.1 | 39.8 |
| | | 11 | 24.2 | 36.1 | 39.6 |

EXAMPLE 7

Following the procedures of Example 1, 40cc of a physical mixture of Engelhard Clay-24 granules (30g) and 5% palladium-on-carbon granules (10g) was charged to the reactor, and then treated with a liquid feed

of C-5 cut (3000g) containing 13.1 wt% of isoamylene and 48.3 wt% of a mixture of 3-methyl-1-butene, 2-methyl-2-butene and 2-methyl-1-butene, and methanol (640g) over a range of temperature (8O-180°C). Samples of the product effluent were taken on-stream periodically and analyzed by GLC and by GLC-iv. Results are summarized in Table VII.

An analysis of the data from Sample 6 shows:

| | |
|---|---|
| Concentration of TAME in the crude effluent | 8.9% |
| Apparent isoamylene conversion | 22% |
| TAME selectivity (basis isoamylene) | 264 mole% |

## TABLE VII

| | | | Liquid Composition (%) | | | | | | | | | |
|---|---|---|---|---|---|---|---|---|---|---|---|---|
| | | | C-4 Olefins | | | | | C-5 Fraction | | | Total Sample | |
| Temp. (°C) | Flow (cc/h) | Sample | 1- | ISO- | CIS-2 | TRANS-2 | $C_4^{2-}$ | 3-Me | 2-Me-2 | 2-Me-1 | MeOH | TAME |
| | | F-1 | 0.6 | 0.4 | 2.4 | 4.1 | 0.1 | 30.4 | 13.1 | 4.8 | 21.2 | - |
| 80 | 80 | 1 | 0.6 | 0.4 | 2.5 | 4.3 | 0.1 | 31.4 | 12.7 | 3.8 | ? | -1.2 |
| | | 2 | 0.6 | 0.3 | 2.5 | 4.3 | 0.1 | 31.6 | 12.7 | 3.7 | 19.9 | 1.7 |
| 100 | 80 | 3 | 0.7 | 0.1 | 2.6 | 4.4 | 0.1 | 33.1 | 10.9 | 2.0 | 16.8 | 6.8 |
| | | 4 | 0.7 | 0.2 | 2.6 | 4.5 | | 32.9 | 10.9 | 2.0 | 16.7 | 7.3 |
| 120 | 80 | 5 | 0.7 | 0.1 | 2.6 | 4.5 | | 33.9 | 10.2 | 1.5 | 16.0 | 8.7 |
| | | 6 | 0.7 | 0.1 | 2.7 | 4.6 | 0.1 | 33.7 | 10.2 | 1.5 | 16.1 | 8.9 |
| | | F-2 | 0.6 | 0.4 | 2.4 | 4.2 | 0.1 | 30.6 | 13.1 | 4.8 | 19.5 | - |
| 140 | 80 | 7 | 0.7 | 0.1 | 2.8 | 4.8 | | 32.7 | 10.8 | 1.6 | 15.8 | 7.9[b] |
| | | 8 | 0.7 | 0.1 | 2.7 | 4.7 | | 32.8 | 10.8 | 1.6 | 16.0 | 7.7 |
| 160 | 80 | 9 | 0.7 | 0.2 | 2.6 | 4.6 | | 29.9 | 12.9 | 2.5 | 13.8 | 4.5 |
| | | 10 | 0.7 | 0.2 | 2.5 | 4.4 | | 30.8 | 13.1 | 2.7 | 17.3 | 3.9 |
| 180 | 80 | 11 | 0.7 | 0.2 | 2.4 | 4.3 | | 29.3 | 13.5 | 2.4 | 11.4 | 3.2[c] |
| | | 12 | 0.7 | 0.3 | 2.5 | 4.4 | | 30.3 | 13.3 | 2.6 | 19.5 | 2.3 |

## EXAMPLE 8

Following the procedures of Example 1, a sample of palladium-on an aminosilated montmorillonite clay (40cc) prepared according to the procedure of Example B, was treated with a mixture of methanol and isoamylene diluted with n-pentane over a range of temperatures (60-140°C). Samples of the product effluent were taken on-stream and analyzed by GLC. Results are summarized in Table VIII.

An analysis of the data from Sample 7 shows:

| | |
|---|---|
| Isoamylene conversion | 41% |
| TAME selectivity (basis isoamylene) | 79% |

TABLE VIII

| Temp. (°C) | Flow (cc/h.) | Sample | PRODUCT COMPOSITION (%) MeOH | $C_5$ | $C_5^-$ | TAME |
|---|---|---|---|---|---|---|
| | | F-1 | 9.4 | 69.7 | 17.1 | - |
| 60 | 80 | 1 | 8.5 | 71.8 | 17.2 | 0.1 |
| | | 2 | 8.3 | 70.7 | 16.9 | 0.1 |
| 80 | 80 | 3 | 9.1 | 71.1 | 15.7 | 1.2 |
| | 80 | 4 | 10.9 | 71.0 | 14.8 | 1.3 |
| 100 | 80 | 5 | 10.5 | 68.4 | 11.2 | 6.3 |
| | 80 | 6 | 10.1 | 69.2 | 12.4 | 5.3 |
| 120 | 80 | → 7 | 7.7 | 69.1 | 10.1 | 8.1 |
| | 80 | 8 | 8.2 | 68.6 | 10.1 | 7.7 |
| 140 | 80 | 9 | 9.8 | 68.8 | 11.4 | 5.0 |
| | 80 | 10 | 9.7 | 69.4 | 11.6 | 4.5 |

EXAMPLE C

This example illustrates the synthesis of a 12-tungstophosphoric acid on montmorillonite clay catalyst for use in Examples 9 and 10.

To a sample of Englehard Grade-24 granules (100g) was added 1 litre of an aqueous solution containing 288g of 12-tungstophosphoric acid. The mixture was stirred mechanically for 2 days at ambient temperature and the solids were recovered by filtration, washed with distilled water until no tungsten could be detected in the filtrate, and then dried in vacuo for 1-2 hours at 40°C.

The finished clay (99g) was found to contain 1.1% of tungsten.

EXAMPLE 9

The synthesis were conducted in the tubular reactor described in Example 1.

The reactor was charged at the beginning of the experiment with 40 cc of the 12-tungstophosphoric acid-on-montmorillonite clay catalyst described in Example C. A screen of glass beads was placed at the top and bottom of the reactor to ensure that the granules would remain in the middle portion. A reactant solution comprising methanol (640g, 20 mole) and a C-5 cut from a light olefins unit (3000g) containing ca. 11.5 wt% isoamylene (4.9 mole) and ca. 40% total of 3-methyl-1-pentene, 2-methyl-2-pentene and 2-methyl-1-pentene, was then pumped through the catalyst bed at 80 cc/h, while the reactor was held at 100°C and a total pressure of 2.17 MPa (300 psi). Samples of the product were taken periodically, by collecting on-stream in a stainless steel pressure bomb. Typical glc analyses data for samples taken under these conditions are summarized in Table IX. For Sample 4, it is estimated that:

2-methyl-2-butene conversion per pass = 35%

Methanol conversion per pass = 44%

TAME selectivity (basis isoamylene converted) = 232 mole%

Catalyst performance over a range of temperatures (80-120°C) and flow rates (40-80 cc/h) was also measured, after reaching equilibrium conditions. Summary data for these additional runs are also given in Table IX.

TAME may be isolated from said typical product effluents in more than 99% purity by fractional distillation.

TABLE IX

| Temp. (°C) | Flow (cc/h) | Sample | Liquid Composition (%) C-5 Fraction C-4 Olefins 1- | Iso | CIS-2 | TRANS-2 | C4- | 3-Me-1 | 2-Me-2 | 2-Me-1 | Total Sample MeOH | MTBE | TAME |
|---|---|---|---|---|---|---|---|---|---|---|---|---|---|
| | | F-1 | 1.5 | 0.9 | 4.1 | 7.6 | | 26.0 | 11.5 | 2.4 | 25.4 | 0.2 | 0.4 |
| 80 | 80 | 1 | 1.4 | 0.7 | 4.2 | 7.7 | 0.2 | 27.0 | 11.1 | 2.5 | 7.2 | 0.5 | 1.7 |
| | | 2 | 1.5 | 0.3 | 4.3 | 8.1 | 0.1 | 27.8 | 10.1 | 2.6 | 14.6 | 1.1 | 5.2 |
| 100 | 80 | 3 | 1.6 | 0.3 | 4.5 | 8.4 | 0.2 | 29.5 | 7.6 | 2.7 | 13.9 | 1.5 | 9.9 |
| | | 4 | 1.6 | 0.1 | 4.6 | 8.6 | 0.2 | 29.3 | 7.5 | 2.7 | 14.2 | 1.5 | 10.3 |
| 120 | 80 | 5 | 1.6 | 0.2 | 4.5 | 8.4 | 0.1 | 28.2 | 9.3 | 2.7 | 14.8 | 1.5 | 7.4 |
| | | 6 | 1.6 | 0.2 | 4.5 | 8.4 | 0.1 | 28.1 | 9.4 | 2.7 | 14.9 | 1.6 | 7.1 |
| 80 | 80 | 7 | 1.5 | 0.1 | 4.4 | 8.2 | 0.2 | 28.7 | 8.9 | 2.7 | 15.2 | 1.3 | 7.9 |
| | | 8 | 1.6 | 0.1 | 4.5 | 8.3 | 0.2 | 28.5 | 8.9 | 2.7 | 15.2 | 1.3 | 8.4 |
| 100 | 40 | 9 | 1.6 | 0.1 | 4.5 | 8.4 | 0.1 | 29.1 | 7.5 | 2.7 | 15.5 | 1.5 | 10.3 |
| | | 10 | 1.6 | 0.1 | 4.5 | 8.5 | 0.2 | 29.3 | 7.6 | 2.7 | 14.3 | 1.5 | 10.3 |

EXAMPLE 10

Following the procedures of Example 9, 40cc of the 12-tungstophosphoric acid-on-montmorillonite clay catalyst described in Example C and containing 0.9 wt% W was treated with a mixture of C-5 cut (3000g) containing about 11.9% isoamylene (5.1 mole) and 41% total of 3-methyl-1-pentene, 2-methyl-2-pentene and 2-methyl-1-pentene, in addition to methanol (640g), at 80°C, using a flow rate of 80 cc/h. Samples of the product effluent were taken on-stream periodically and analyzed by glc for C-5 cut and TAME/MeOH content. Results are summarized in Table X.

Catalyst performance was checked over a range of operating temperatures (80-140°C). Summary data for these additional runs may also be found in Table X. For Sample 5 at 120°C:
2-methyl-2-butene conversion per pass = 14%
Methanol conversion per pass = 14%
TAME selectivity (basis isoamylene converted) = 261 mole%

TABLE X

| | | | Liquid Composition (%) | | | | | | | | | | |
|---|---|---|---|---|---|---|---|---|---|---|---|---|---|
| | | | C-4 Olefins | | | | | C-5 Fraction | | | Total Sample | | |
| Temp. (°C) | Flow (cc/hr) | Sample | 1- | Iso | CIS-2 | TRANS-2 | C4- | 3-Me-1 | 2-Me-2 | 2-Me-1 | MeOH | MTBE | TAME |
| | | F-1 | 1.2 | 1.0 | 3.6 | 6.6 | 0.1 | 27.3 | 11.9 | 2.5 | 18.2 | | |
| 80 | 80 | 1 | 1.2 | 0.8 | 3.7 | 6.8 | 0.1 | 27.6 | 11.7 | 2.5 | 17.6 | 1.2 | 1.4 |
| | | 2 | 1.2 | 0.7 | 3.7 | 6.8 | 0.1 | 27.9 | 11.4 | 2.5 | 16.8 | 0.9 | 2.5 |
| 100 | 80 | 3 | 1.2 | 0.5 | 3.8 | 7.0 | 0.1 | 28.6 | 10.8 | 2.6 | 15.4 | 1.4 | 4.5 |
| | | 4 | 1.3 | 0.5 | 3.9 | 7.0 | 0.2 | 28.4 | 10.8 | 2.6 | 16.3 | 1.7 | 4.7 |
| 120 | 80 | 5 | 1.3 | 0.3 | 3.9 | 7.1 | 0.2 | 29.1 | 10.2 | 2.7 | 15.7 | 2.0 | 6.4 |
| | | 6 | 1.3 | 0.4 | 3.9 | 7.1 | 0.2 | 29.0 | 10.3 | 2.6 | 15.6 | 1.9 | 6.2 |
| 140 | 80 | 7 | 1.2 | 0.4 | 3.8 | 7.0 | | 28.7 | 11.8 | 2.6 | 16.9 | 1.1 | 3.4 |
| | | 8 | 1.2 | 0.4 | 3.8 | 7.0 | | 28.7 | 11.8 | 2.6 | 16.9 | 1.1 | 3.4 |
| 120 | 40 | 9 | 1.2 | 0.3 | 3.8 | 6.8 | | 29.4 | 10.2 | 2.7 | 16.7 | 1.7 | 6.5 |
| | | 10 | 1.3 | 0.3 | 3.9 | 7.1 | | 29.1 | 10.1 | 2.7 | 16.2 | 1.3 | 6.4 |

## Claims

**1.** A method for the preparation of tertiary amyl methyl ether comprising reacting methanol and C-5 olefin over an acidic smectite clay catalyst at a temperature of 20 to 250°C and a pressure of 0.1 to 7.0 MPa, characterized in that the catalyst is modified by palladium or by a heteropoly acid.

**2.** A method according to Claim 1 characterized in that the smectite clay is an acidic montmorillonite silica-alumina clay having the formula:

$$M^{n+}_{x/n} \bullet yH_2O(Al_{4-x}Mg_x)(Si_8)O_{20}(OH)_4$$

wherein M represents the interlamellar balancing sodium or lithium cations, x, y and n are numbers, and the acidity of the clay is 3 to 20 mg KOH/gm.

**3.** A method according to Claim 1 or 2 characterized in that the acidic smectite clay is pretreated with a mineral acid.

**4.** A method according to Claim 3 characterized in that a heteropoly acid is bonded to the acidic montmorillonite silica-alumina clay.

**5.** A method according to Claim 4 characterized in that the heteropoly acid is 12-tungstophosphoric acid, 12-molybdophosphoric acid, tungstosilicic acid or 12-molybdosilicic acid.

**6.** A method according to Claim 4 or 5 characterized in that the concentration of the polyatom in the formulated catalyst is from 0.1 to 20 wt%.

**7.** A method according to any one of Claims 1 to 3 characterized in that the acidic smectite clay is modified with palladium.

**8.** A method according to Claim 7 characterized in that the palladium-modified smectite clay is prepared by ion-exchange of a palladium salt with the smectite clay.

**9.** A method according to Claim 7 characterized in that the palladium-modified smectite clay is prepared by adding a palladium salt to an aminosilated smectite clay.

**10.** A method according to any one of Claims 1 to 3 characterized in that the acidic clay catalyst is physically mixed with a supported palladium catalyst.

**11.** A method according to any one of Claims 1 to 10 characterized in that the C-5 olefin is isoamylene.

**12.** A method according to any one of Claims 1 to 10 characterized in that the C-5 olefin is a mixture of 3-methyl-1-butene, 2-methyl-2-butene and 2-methyl-1-butene.

**13.** A method according to any one of Claims 1 to 10 characterized in that the C-5 olefin is from a raffinate stream from a methyl tertiary butyl ether production unit.

**14.** A method according to any one of Claims 1 to 10 characterized in that the C-5 olefin is a C-5 cut from a light olefins unit.

**15.** A method according to any one of Claims 1 to 14 characterized in that a saturated hydrocarbon or ether diluent is employed.

European Patent Office

# EUROPEAN SEARCH REPORT

Application Number: EP 91301079.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE - A1 - 3 813 689 (DEUTSCHE BP AG) * Claims 1-6,8 * -- | 1,2, 7-11 | C 07 C 43/04<br>C 07 C 41/06 |
| Y | EP - A1 - 0 249 352 (THE BRITISH PETROLEUM COMPANY) * Claims 1,3-5,8-10 * -- | 1,2 | |
| Y | EP - A1 - 0 333 076 (TEXACO DEVELOPMENT CORPORATION) * Claims 1-3,8,9 * -- | 1,5 | |
| Y | US - A - 4 259 533 (ANDREW T. GUTTMANN et al.) * Claims 1-3 * -- | 1,2,5 | |
| Y | EP - A1 - 0 333 078 (TEXACO DEVELOPMENT CORPORATION) * Claims 1,5,6 * -- | 1-4 | TECHNICAL FIELDS SEARCHED (Int. Cl.5) |
| A | EP - A1 - 0 256 185 (EXXON RESEARCH) * Claim 1; page 8, lines 17-34 * -- | 1 | C 07 C 43/00<br>C 07 C 41/00<br>B 01 J |
| A | GB - A - 2 179 563 (THE BRITISH PETROLEUM COMPANY) * Claims 1,2,10,14 * -- | 1,2 | |
| A | US - A - 4 299 998 (PAUL R. STAPP) * Abstract * -- | 1,10, 11 | |
| A | US - A - 4 175 210 (CHARLES M. SELWITZ et al.) * Abstract * ---- | 1,5 | |

The present search report has been drawn up for all claims

| Place of search | Date of completion of the search | Examiner |
|---|---|---|
| VIENNA | 11-07-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document